# EUROPEAN PATENT SPECIFICATION

(11) **EP 1 935 440 B1**
(45) Date of publication and mention of the grant of the patent: **28.04.2010**
(21) Application number: 07123244.1
(22) Date of filing: 14.12.2007
(51) Int. Cl.: A61M 1/00

(54) **System and method for controlling fluid flow in an aspiration chamber**
System und Verfahren zur Regelung des Fluidflusses in einer Absaugkammer
Système et procédé de contrôle de débit de fluide dans une cuve d'aspiration

(30) Priority: 18.12.2006 US 612214
(43) Date of publication of application: 25.06.2008
(73) Proprietor: Alcon, Inc., 6331 Hünenberg (CH)
(72) Inventor: Hopkins, Mark Alan, Mission Viejo, CA 92691 (US); Gao, Shawn X., Irvine, CA 92620 (US)
(74) Representative: Hanna, Peter William Derek

(56) References cited:
- EP-A- 1 839 689
- WO-A-93/18802
- WO-A-2007/001503
- US-A- 4 773 897
- US-A1- 2007 219 494

## Description

### TECHNICAL FIELD OF THE INVENTION

The present invention relates to a surgical console.

### BACKGROUND OF THE INVENTION

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreoretinal surgery, anterior segment procedures, such as cataract surgery, and combined anterior and posterior segment procedures.

The surgical instrumentation used for ophthalmic surgery can be specialized for posterior segment procedures or anterior segment procedures or support both. In any case, the surgical instrumentation often requires the use of associated consumables such as surgical cassettes, fluid bottles I bags, tubing, hand piece tips and other consumables.

A surgical cassette can provide a variety of functions depending on the procedure and surgical instrumentation. For example, surgical cassettes for cataract surgeries help manage irrigation and aspiration flows into and out of a surgical site. Surgical cassettes provide an interface between surgical instrumentation and the patient, delivering pressurized infusion and aspiration flows into and out of the eye. For a typical venturi based aspiration system, the fluid is first extracted from a patient eye into an aspiration chamber, and then removed from the aspiration chamber into a drain bag.

The aspiration chamber can serve many functions. For example, the aspiration chamber can function as a sensing chamber for a continuous level sensing, which provides volumetric and aspiration flow information to the surgical console. The aspiration fluid generally enters the aspiration chamber from the top. Due to dripping, the aspiration fluid entering from the top of the aspiration chamber unavoidably causes disturbance to the fluid surface and consequently affects the continuous level sensing. Moreover, when the fluid port is at the top of the chamber, not all of the fluid can be used for reflux procedures. Consequently, new solutions are needed to minimize the disturbance to the liquid/air surface in the aspiration chamber and to make more fluid available for reflux. Previously, to conduct a reflux procedure a mechanical force is applied to the aspiration line to force the liquid out of the handpiece. The forced backflow of liquid (i.e., the aspiration fluid travels in the opposite direction to its normal movement) may or may not successfully unblock or unclogged the handpiece. Moreover, because the aspiration port is positioned at the top of the aspiration chamber, the forced out liquid is not necessarily replaced by more liquid. Therefore, a new solution is needed to make the reflux operation sustainable. Embodiments of the invention disclosed herein can address these needs and more.

Document WO 93/18802 discloses a surgical console comprising a cassette-receiving portion and a cassette. The cassette comprises an aspiration chamber with an aspiration port located at the bottom of the aspiration chamber. The console comprises a photoelectric cell for sensing the volume of fluid in the aspiration chamber.

### SUMMARY OF THE INVENTION

The present invention provides a surgical cassette received in a surgical console for controlling the flow of aspiration fluid in a surgical cassette, in accordance with claims which follow. When the aspiration fluid enters the aspiration chamber of the surgical cassette, the disturbance to the liquid/air surface must be minimized so as to facilitate the many functions of the aspiration chamber. For example, through a continuous level sensing the aspiration chamber functions as a sensing chamber and provides volumetric and aspiration flow information to the surgical console. The aspiration chamber also functions as a fluid reservoir for sustained reflux and priming.

Embodiments of the invention address these needs by placing the aspiration port at or close to the bottom of the aspiration chamber. When the aspiration fluid enters the aspiration chamber from below the fluid surface (e.g., from a port located at the bottom or on a sidewall close to the bottom), it does not create disturbance at the liquid/air interface and thus minimizes any effect on the continuous level sensing. Also, since the aspiration port is placed at or very close to the bottom of the aspiration chamber, the liquid inside the chamber can be fully used.

Embodiments of the present invention provide many advantages over prior art systems of aspiration flow control in surgical cassettes. For example, by placing the aspiration port at the bottom of the aspiration chamber, it eliminates the disturbances to the fluid surface when the aspiration fluid enters the aspiration chamber. As a result, it minimizes the noise introduced to the continuous level sensing and improves the signal to noise ratio, which leads to more accurate and reliable data needed to determine the fluid to air ratio changes over time and flow rate of aspiration fluid into and out of the aspiration chamber.

Another advantage provided by embodiments of the invention is directed to the reflux and priming of the aspiration line. In embodiments where the aspiration port is positioned at the bottom of the aspiration chamber, the liquid in the chamber can be fully used for reflux and priming. This makes it possible for sustained reflux and pushing priming.

### BRIEF DESCRIPTION OF THE DRAWINGS

A more complete understanding of the present invention and the advantages thereof may be acquired by referring to the following description, taken in conjunction with the accompanying drawings in which like reference numbers indicate like features and wherein:
FIGURE 1 is a diagrammatic representation of one embodiment of a surgical console;
FIGURE 2 is a diagrammatic representation of a surgical cassette;
FIGURE 3 is a diagrammatic representation of a cassette receiver;
FIGURE 4 is a diagrammatic representation of a system for continuous sensing of fluid and air volumes in an aspiration chamber of a surgical cassette;
FIGURE 5 is a diagrammatic representation of a system for controlling the fluid and air volumes in an aspiration chamber of a surgical cassette;
FIGURE 6 is a diagrammatic representation of a system implementing an aspiration chamber with an aspiration port positioned at the bottom of the aspiration chamber.

### DETAILED DESCRIPTION

Preferred embodiments of the invention are illustrated in the FIGURES, like numerals being used to refer to like and corresponding parts of the various drawings.

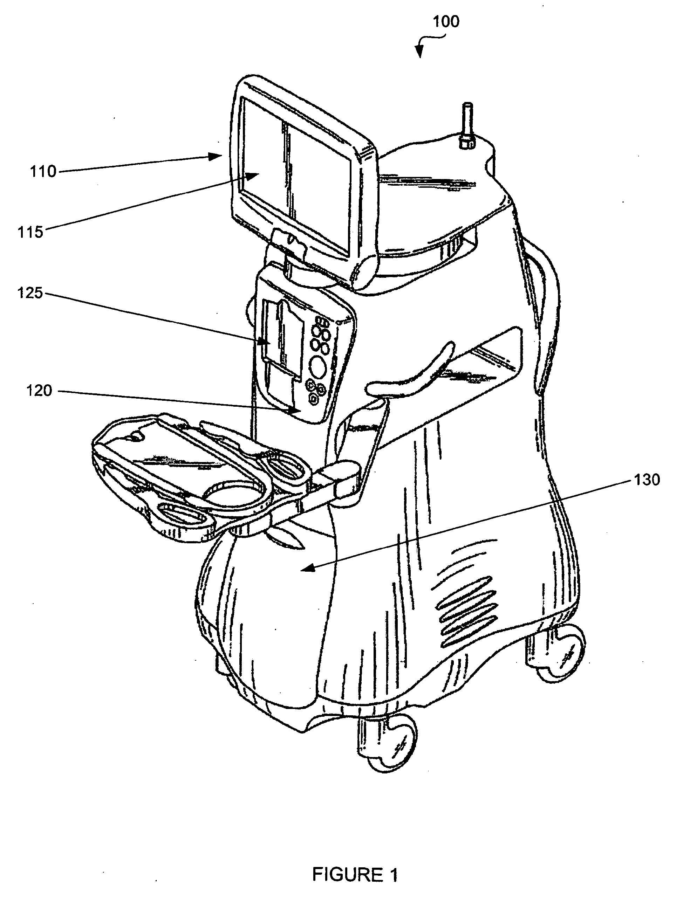

FIGURE 1 is a diagrammatic representation of one embodiment of an ophthalmic surgical console 100. Surgical console 100 can include a swivel monitor 110 that has touch screen 115. Swivel monitor 110 can be positioned in a variety of orientations for whomever needs to see touch screen 115. Swivel monitor 110 can swing from side to side, as well as rotate and tilt. Touch screen 115 provides a graphical user interface ("GUI") that allows a user to interact with console 100.

Surgical console 100 also includes a connection panel 120 used to connect various tools and consumables to surgical console 100. Connection panel 120 can include, for example, a coagulation connector, connectors for various hand pieces, and a cassette receiver 125. Surgical console 100 can also include a variety of user friendly features, such as a foot pedal control (e.g., stored behind panel 130) and other features.

In operation, a cassette (not shown) can be placed in cassette receiver 125. A clamp in surgical console 100 clamps the cassette in place to minimize movement of the cassette during use. The clamp can clamp the top and bottom of the cassette, the sides of the cassette or otherwise clamp the cassette.

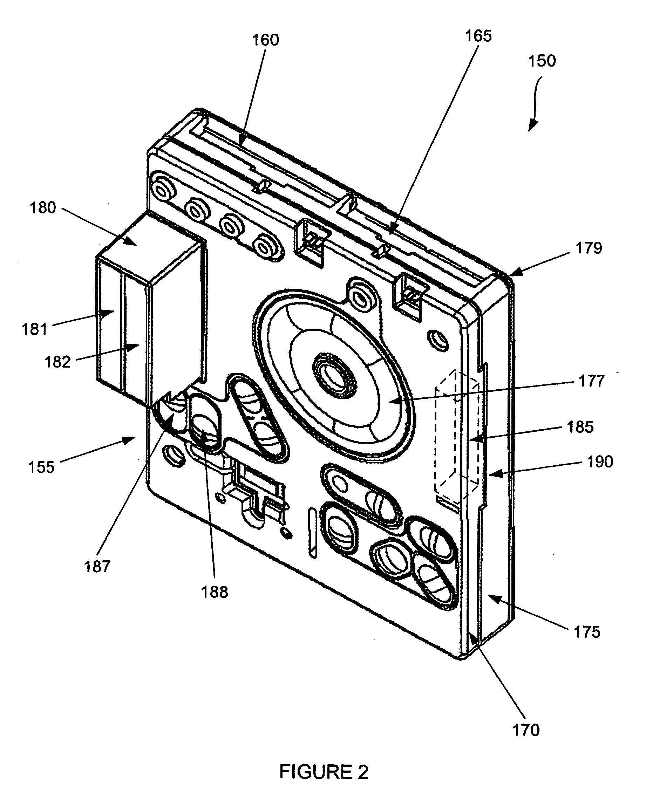

FIGURE 2 is a diagrammatic representation of a surgical cassette 150. Cassette 150 can provide a closed system fluidic device that can be discarded following a surgical procedure. Cassette 150 can include a cassette body 155 and portions that interface with the clamp (e.g., indicated generally at clamping zones 160 and 165) projecting from the cassette body 155 Cassette 150 can be formed of ABS plastic or other suitable material. In the example shown, cassette 150 is formed from three primary sections: an inner or surgical console interface section 170 that faces the surgical console when cassette 150 is inserted into surgical console 100, a middle section 175 and a cover plate 179. The various sections of cassette 150 can be coupled together via a press fit, interlocking tabs, chemical bonding, thermal bonding, mechanical fasteners or other attachment mechanism known in the art. In other examples, cassette 150 can be formed of a single piece or multiple pieces.

Surgical console interface section 170 can face the console during use and provide an interface for fluid flow channels (e.g., flow channel 177 for the peristaltic pump provided by an elastomeric pump membrane), valves (e.g., infusion/aspiration valves), and other features to manage fluid flow. Cassette 150 can also attach to a fluid bag (not shown) to collect fluids during a procedure.

Surgical cassette 150 includes chambers to hold fluids for aspiration and infusion. For example, chamber cartridge 180 can include two chambers 181 and 182. A third chamber 185 can be internal to cassette 150 on the opposite side of cassette 150 from chamber cartridge 180 (e.g., at the side of cassette 150 indicated by 190). In one embodiment, chambers 181 and 182 are infusion chambers and chamber 185 is an aspiration chamber. Valve seals 187/188 can be formed of separate pieces or a single piece of an elastomeric material and can return to approximately their original shapes when the forces applied by the actuators are removed. Different valves in cassette 150 can have the same or different configurations to control the flow through the cassette.

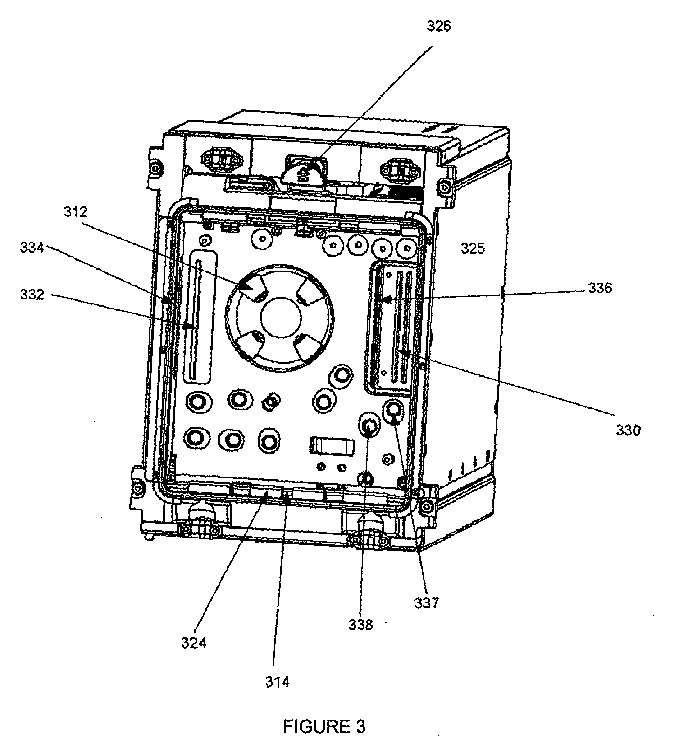

FIGURE 3 is a diagrammatic representation of cassette receiver 325 for receiving a surgical cassette (not shown). Cassette receiver 325 can have various pneumatic input and output ports to interface with the surgical cassette. Cassette receiver 325 can further include an opening to allow peristaltic pump rollers 312 to contact the surgical cassette during operation. One example of a peristaltic pump and complimentary cassette is described in United States Patent No. 6,293,926 to Sorensen.

The surgical cassette is held in place by a clamp having a bottom rail 314 and a top rail (not shown). Each rail can have outer clamping fingers (e.g., clamp finger 324) that contact the cassette in corresponding clamping zones and inner clamping fingers to locate the cassette during insertion and push the cassette out of cassette receiver during release. A release button 326 is pressed to initiate release of the cassette from the clamp. Cassette receiver 325 can include linear light sources to project light into the walls of the cassette chambers and sensor arrays to detect the light refracted through the chamber (or reflected from the chamber wall). Each linear light source can include a plurality of light sources vertically arranged (i.e., to project light along vertically spaced transmission paths) and positioned to project light into a wall of the cassette. For example, linear light source 332 can project light into the walls of the aspiration chamber 185. Respective linear sensor arrays can receive light refracted through the chamber or reflected at the chamber surface. Each sensor array can include vertically arranged portions to receive light through the wall of the cassette chamber. The vertically arranged portions can be, for example, pixel sensors, separate sensors or other mechanisms for sensing illumination.

As described in our EP-1,873,501-A, entitled "System and Method of Non-Invasive Continuous Level Sensing," the level and hence volume of fluid in a chamber can be determined by projecting light into the wall of the cassette and evaluating the light pattern detected by the corresponding linear sensor array. By tracking the change in volume over time, the volumetric or mass flow rate of fluid into/out of the chamber can be determined. As noted above, the flow rate of fluid into a chamber can be regulated by a vacuum source, e.g. a Venturi Pump in the surgical console.

The configuration of FIGURE 3 is provided by way of example. The form factor of cassette receiver 325, placement and number of input/output ports and other features of cassette receiver 325 can depend on the surgical console 100, surgical procedure being performed or other factors.

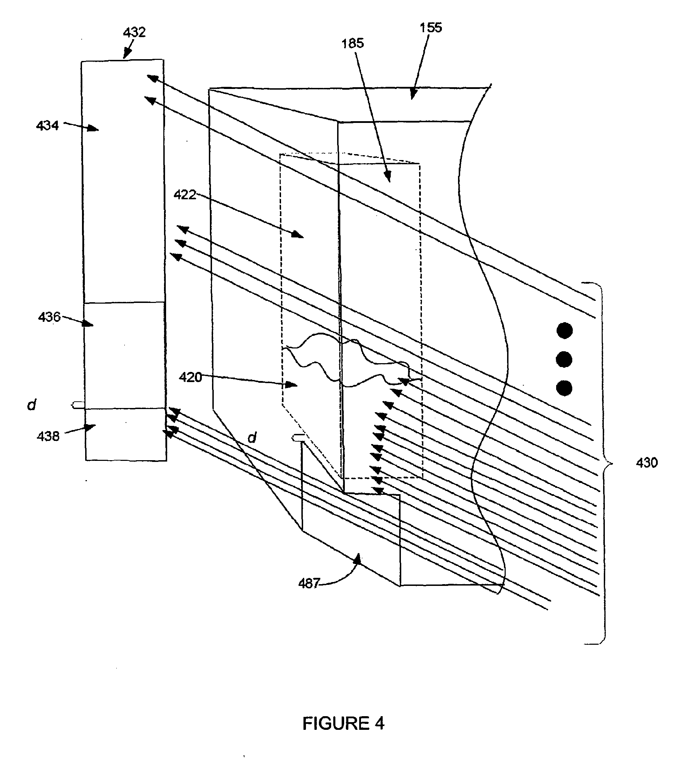

FIGURE 4 is a diagrammatic representation of a system for determining the volumetric or mass flow rate of fluid into/out of a chamber (e.g., an aspiration chamber). In this example, the bottom of aspiration chamber 185 is filled with fluid 420 while the top of aspiration chamber 185 contains air 422. A linear light source projects light (represented by light rays 430). As illustrated in FIGURE 4, a portion of light rays 430 can pass through cutout 487 unobstructed while other portions of light rays 430 travel through various combinations of media having different optical properties (e.g., fluid 420, air 422, aspiration chamber 185, cassette body 155, etc.) As one skilled in the optical art can appreciate, when light travels from one medium to another, it bends and changes directions (i.e., reflect or refract) at the interface between two different materials. The amount of bending can depend on the angle of incidence as well as the optical property (e.g., refraction index) of each material. A linear sensor array having vertically arranged portions as described above is positioned to receive light rays 430. The illumination pattern 432 illustrates the result of directing light rays 430 at aspiration chamber 185. As an example, illumination pattern 432 comprises a first area 434 illuminated by light rays 430 passing through aspiration chamber 185 and air 422 inside, a second area 436 insufficiently illuminated (e.g., illuminated below a threshold value) by light rays 430 encountering aspiration chamber 185, fluid 420. In this way, the upper level of fluid 420 can be determined based on the transition between area 434 (e.g., the upper section of pixels in the "ON" state) and area 436 (e.g., pixels in the "OFF" state).

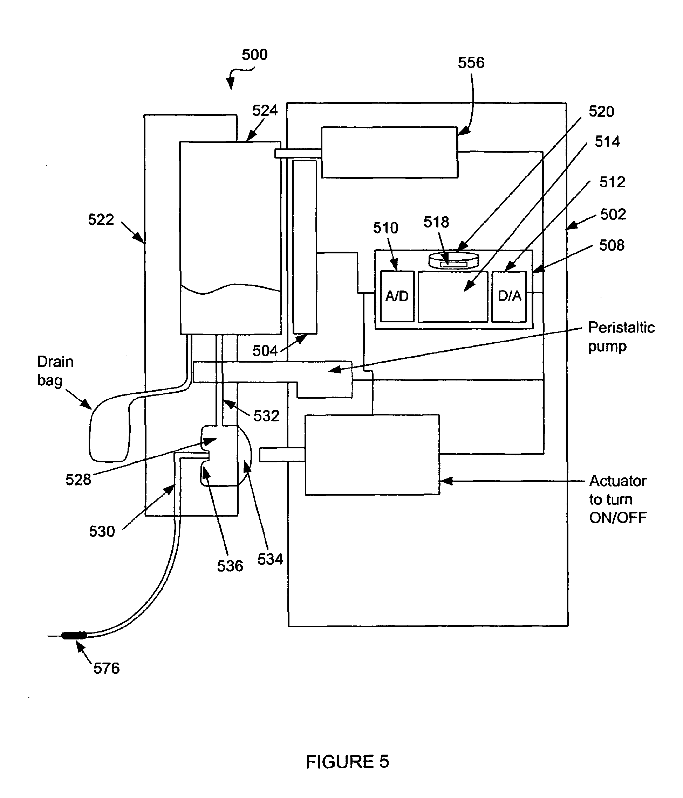

FIGURE 5 is a diagrammatic representation of a flow control mechanism embodied in a surgical system. In the example shown in FIGURE 5, surgical system 500 includes a surgical console 502 having a sensor system 504, a pneumatic pressure/vacuum source 556, and a controller 508. Controller 508 can be any suitable controller known in the art including DSP, ASIC, RISK or CPU based controllers. Controller 508 can include an analog to digital (AID) converter 510 to convert analog signals from sensor system 504 to digital signals. Additionally, controller 508 can include a digital to analog (D/A) converter 512 to convert digital control signals to analog signals to control sensor system 504 and pneumatic pressure/vacuum source 656. A processor 514, such as a DSP, ASIC, RISC, microcontroller or CPU or other suitable processor can access a set of instructions 518 on a computer readable memory 520. The computer readable memory 520 can be RAM, ROM, magnetic storage, optical storage or other suitable memory and can be onboard or be accessible by processor 514.

Surgical system 500 can further include surgical cassette 522 inserted into surgical console 502. Surgical cassette 522 can include a fluid chamber 524, such as an infusion chamber or aspiration chamber that can act as a fluid reservoir for surgical instrumentation. Fluid from a hand piece 576 is led to a valve chamber 528 via an inlet flow passage 530 and from valve chamber 528 to fluid chamber 524 via an outlet flow passage 532. The flow rate of fluid flowing from the hand piece 676 to fluid chamber 524 is controlled by the pneumatic pressure/vacuum source 556.

Controller 508 can implement various control schemes known or developed in the art to generate signals to control the pneumatic pressure/vacuum source 556 based on a comparison of a measured flow rate and a set point flow rate. In one embodiment, chamber 524 is an aspiration chamber connected to a venturi-based pressure/vacuum system, 556 which works in concert with controller 508 to supply suction pressure to the aspiration chamber. In this case, aspiration chamber is connected to a handpiece via an aspiration line. Aspiration fluid is extracted by suction from a surgical site via the handpiece and sent to the aspiration chamber through the aspiration line.

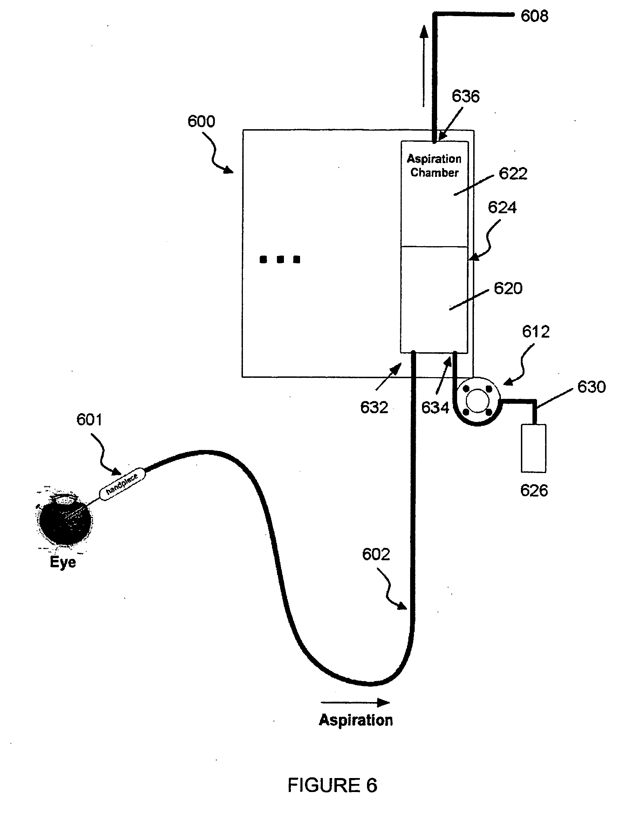

FIGURE 6 is a diagrammatic representation of a system implementing an aspiration chamber with an aspiration port positioned at the bottom of the aspiration chamber. In one example, surgical cassette 600 comprises two infusion chambers (not shown) and one aspiration chamber 624. The dual infusion chambers design allows one to be refilled while the other one is in use. During a surgical procedure, pressurized liquid (e.g., an irrigating solution) is pushed out from the infusion chamber to the surgical site (e.g., a patient eye) and extracted (via handpiece 601 and aspiration line 602) from the patient eye to aspiration chamber 624 and then removed via drain port 634 and fluidic channel 630 from the aspiration chamber into a drain bag 626. Fluidic channel 630 may be embedded in surgical cassette 600 and may comprise an elastic cover or soft membrane covering. Drain pump or actuator 612 may operate to compress or otherwise apply force onto fluidic channel 630, causing a positive displacement and forcing aspiration fluid 620 to move into drain bag 626. In one example, drain pump 612 may be a peristaltic pump utilizing rollers (e.g., rollers 312 of FIGURE 3) to compress the elastic membrane of the flow channel. The aspiration flow rate can be flow controlled or vacuum controlled.

In one example, aspiration chamber 624 is connected to a venturi-based pressure/vacuum system 608 through port 636. In one example, pressure/vacuum system 608 resides in surgical console. In one embedment, pressure/vacuum system 608 provides aspiration pressure or vacuum. One example of a venturi-based vacuum system is the ACCURUS® system from Alcon Laboratories, Inc. In conjunction with an aspiration pressure system, a controller such as controller 608 as described above with reference to FIGURE 5 or other suitable control circuitry can be implemented to control the aspiration pressure and aspiration fluid flow.

Previously, the aspiration chamber is fitted with an aspiration port on the top. When the aspiration fluid extracted from the surgical site enters into the aspiration chamber through the aspiration port, it drips from the top and causes disturbance to the fluid surface in the aspiration chamber. To eliminate this problem and minimize the disturbance to the liquid/air surface, aspiration chamber 624 is designed to have an aspiration port 632 located at the bottom of aspirating chamber 624, allowing aspiration fluid 620 from aspiration line 602 to enter aspiration chamber 624 from below the surface of fluid 620. This bottom-entry configuration eliminates dripping and thus the disturbance to the continuous level sensing function described above with reference to FIGURE 4, allowing aspiration chamber 624 to provide significantly more accurate volumetric and aspiration flow information to the surgical console.

In one example, aspiration port 632 is positioned close to the bottom (e.g., on a sidewall) of aspiration chamber 624. When aspiration fluid 620 enters aspiration chamber 624, it again enters from below the fluid surface and does not affect the continuous level sensing. As a result, it minimizes the noise introduced to the continuous level sensing, allowing aspiration chamber 624 to provide more accurate and reliable data needed to determine the fluid to air ratio changes over time.

Having the aspiration fluid entry located at or close to the bottom of an aspiration chamber has additional advantages. For example, since the aspiration port is placed at or very close to the bottom of the aspiration chamber, the liquid inside the chamber can be fully used. More particularly, the aspiration chamber also functions as a fluid reservoir for sustained reflux and push priming, where the liquid inside the aspiration chamber is pressurized and pushed towards the hand piece to replace the air volume in the aspiration line. Reflux can be achieved by pressurizing the aspiration volume. Since aspiration port 632 is arranged to be at or close to the bottom, air is not introduced into aspiration line 602 during reflux. Priming aspiration line 602 can be similarly done with ease due to the bottom-entry design of aspiration chamber 624.

Furthermore, in some cases, hand piece 601 may be blocked (e.g., due to tissue or other extracted solid materials in aspiration fluid 620) or become restrictive (e.g., due to down scaling). In one embodiment, a control scheme as described below, can be implemented to flush out aspiration fluid 620 via hand piece 601.

For example, the flow rate can be adjusted based on whether the flow rate is outside of some range about the set point. Moreover, the controller can be programmed to perform a flushing operation. For example, if a command or signal is received by the controller indicating a need or desire to flush the aspiration line, the controller can signal the vacuum or similar pressure system connected to the aspiration chamber to increase the air volume inside the aspiration chamber, forcing the fluid out of the aspiration chamber through the aspiration port located at or near the bottom of the aspiration chamber. A push priming operation for the aspiration chamber can be conducted in a similar manner by increasing the air volume inside the aspiration chamber. Since the aspiration port is located at or near the bottom of the aspiration chamber, priming the aspiration line or tube can be easy and efficient as only a minimal amount of fluid is necessary.

## Claims

1. A surgical console (100,502) comprising:
a surgical cassette-receiving portion (125,325), adapted to operably receive a surgical cassette;
a surgical cassette comprising:
an aspiration chamber (185,524,624) for containing an aspiration fluid (420,620) extracted from a surgical site during a surgical procedure;
an aspiration port (632) located at or close to bottom of the aspiration chamber, allowing the aspiration fluid to enter the aspiration chamber from below the fluid surface when the aspiration chamber is at least partially filled;
a pneumatic port (636) located at or close to top of the aspiration chamber, wherein the aspiration fluid inside the aspiration chamber is pressurized by the pneumatic port; and
a drain port (634) located at or close to the bottom of the aspiration chamber;
the surgical console having a linear light source to project light into the aspiration chamber and a linear sensor array system (504) for continuously sensing fluid level inside the aspiration chamber of the cassette, and comprising a controller (508) coupled to the sensor array system and the vacuum system for managing a plurality of functions utilizing the aspiration chamber;
the cassette being adapted to be positioned within the cassette receiving portion such that said fluid surface in the aspiration chamber (624) is measurable by said sensor array system (504) to provide volumetric and aspiration flow information to the surgical console,
the controller (508) being adapted to perform a reflux operation and a priming operation of an aspiration line (602) connecting a handpiece (576,601) and the aspiration port (632) of the cassette, wherein the aspiration port (632) and the drain port (634) are positioned in the aspiration chamber of the cassette to permit fluid in the chamber to be fully used for reflux and priming and wherein the aspiration chamber is configured to function as a fluid reservoir for sustained reflux and push priming without disturbance of the continuous level sensing.

2. The surgical console of Claim 1, further comprising:
a pneumatic system (608) fluidly coupled to the pneumatic port (636) for providing aspiration pressure or vacuum (656) to the aspiration chamber.

3. The surgical console of Claim 1, wherein the controller (508) is operable to manage flow rate of the aspiration fluid based on changes in the fluid level over time in the aspiration chamber.

4. The surgical console of any of Claims 1 to 3, further comprising:
a handpiece (576,601) for extracting the aspiration fluid from the surgical site during the surgical procedure; and
an aspiration line (602) connecting the handpiece and the aspiration port (632).

5. The surgical console of Claim 4, wherein the controller (508) is operable to conduct a sustained reflux operation, forcing liquid out of the handpiece (576,601).

6. The surgical console of Claim 4, wherein the controller (508) is operable to conduct a priming operation for the aspiration line (602).

7. The surgical console of Claim 4, wherein the controller (508) is operable to conduct a priming operation for the aspiration line (602) by causing the vacuum system to supply pressure to the aspiration chamber (624) and replace air in the aspiration line with pressurized liquid from the aspiration chamber.

8. The surgical console of any of Claims 1 to 7, further comprising:
a drain bag (626) for containing a portion of the aspiration fluid; and
a fluidic channel (630) connecting the drain port and the drain bag.

## Patentansprüche

1. Chirurgische Konsole (100, 502) mit:
einem Aufnahmeabschnitt (125, 325) für eine chirurgische Kassette, der zur funktionalen Aufnahme einer chirurgischen Kassette eingerichtet ist,
eine chirurgische Kassette mit:
einer Aspirationskammer (185, 524, 624) zur Aufnahme eines Ansaugfluids (420, 620), das aus einer Operationsstelle während eines chirurgischen Verfahrens extrahiert wird;
einem Ansauganschluss (632), der im oder nahe am Boden der Aspirationskammer angeordnet ist, so dass das Ansaugfluid von unterhalb der Fluidoberfläche in die Aspirationskammer eintreten kann, wenn die Aspirationskammer mindestens teilweise gefüllt ist;
einem Pneumatikanschluss (636), der in oder nahe der Oberseite der Aspirationskammer angeordnet ist, wobei das Ansaugfluid in der Aspirationskammer über den Pneumatikanschluss mit Druck beaufschlagt wird; und
einem Drain-Anschluss (634), der im oder nahe am Bode der Aspirationskammer angeordnet ist; wobei
die chirurgische Konsole eine lineare Lichtquelle, um Licht in die Aspirationskammer zu projizieren, und ein lineares Sensormatrixsystem (504) zur kontinuierlichen Erfassung des Fluidpegels in der Aspirationskammer der Kassette hat, und eine Steuerung (508) aufweist, die mit dem Sensormatrixsystem und dem Vakuumsystem gekoppelt ist, um eine Mehrzahl Funktionen unter Nutzung der Aspirationskammer zu steuern; wobei
die Kassette dazu eingerichtet ist, dass sie im Kassettenaufnahmeabschnitt so positioniert ist, dass die Fluidoberfläche in der Aspirationskammer (624) durch das Sensormatrixsystem (504) messbar ist, um der chirurgischen Konsole Volumen- und Ansaugdurchsatzinformationen bereitzustellen, wobei
die Steuerung (508) dazu eingerichtet ist, eine Rückfluss- und eine Vorfülloperation einer Ansaugleitung (602) auszuführen, die ein Handstück (576, 601) und den Ansauganschluss (632) der Kassette verbindet, wobei der Ansauganschluss (632) und der Drain-Anschluss (634) in der Aspirationskammer der Kassette so positioniert sind, dass das Fluid in der Kammer vollständig für den Rückfluss und das Vorfüllen genutzt werden kann, und wobei
die Aspirationskammer so konfiguriert ist, dass sie als Fluidbehälter für den nachhaltigen Rückfluss und das nachhaltige Druckvorfüllen fungiert, ohne die kontinuierliche Pegelerfassung zu stören.

2. Chirurgische Konsole nach Anspruch 1, ferner aufweisend:
ein Pneumatiksystem (608), das fluidisch mit dem Pneumatikanschluss (636) gekoppelt ist, um die Aspirationskammer mit Ansaugdruck oder Unterdruck (656) zu beaufschlagen.

3. Chirurgische Konsole nach Anspruch 1, bei der die Steuerung (508) so arbeiten kann, dass sie den Durchsatz des Ansaugfluids auf Basis von Änderungen des Fluidpegels über der Zeit in der Aspirationskammer steuert.

4. Chirurgische Konsole nach einem der Ansprüche 1 bis 3, ferner aufweisend:
ein Handstück (576, 601) zum Extrahieren des Ansaugfluids von der Operationsstelle während des chirurgischen Verfahrens; und
eine Ansaugleitung (602), die das Handstück und den Ansauganschluss (632) verbindet.

5. Chirurgische Konsole nach Anspruch 4, bei der die Steuerung (508) so arbeiten kann, dass sie eine nachhaltige Rückflussoperation ausführt, bei der Fluid aus dem Handstück (576, 601) unter Druck ausgestoßen wird.

6. Chirurgische Konsole nach Anspruch 4, bei der die Steuerung (508) so arbeiten kann, dass sie eine Vorfülloperation für die Ansaugleitung (602) ausführt.

7. Chirurgische Konsole nach Anspruch 4, bei der die Steuerung (508) so arbeiten kann, dass sie eine Vorfülloperation für die Ansaugleitung (602) ausführt, indem das Vakuumsystem veranlasst wird, die Ansaugkammer (624) mit Druck zu beaufschlagen und die Luft in der Ansaugleitung mit unter Druck stehender Flüssigkeit aus der Ansaugkammer zu ersetzen.

8. Chirurgische Konsole nach einem der Ansprüche 1 bis 7, ferner aufweisend:
einen Drain-Beutel (626) zur Aufnahme eines Teils des Ansaugfluids; und
einen Fluidkanal (630), der den Drain-Anschluss und den Drain-Beutel verbindet.

## Revendications

1. Console chirurgicale (100, 502) comprenant :
une partie (125, 325) destinée à la réception d'une cassette chirurgicale, adaptée pour recevoir de manière fonctionnelle une cassette chirurgicale ;
une cassette chirurgicale comprenant :
une chambre d'aspiration (185, 524, 624) destinée à contenir un fluide d'aspiration (420, 620) extrait d'un site chirurgical pendant une procédure chirurgicale ;
un orifice d'aspiration (632) situé au niveau ou à proximité du fond de la chambre d'aspiration, qui permet au fluide d'aspiration d'entrer dans la chambre d'aspiration par-dessous la surface du fluide quand la chambre d'aspiration est au moins partiellement remplie ;
un orifice d'accès pneumatique (636) situé au niveau ou à proximité du dessus de la chambre d'aspiration, le fluide d'aspiration à l'intérieur de la chambre d'aspiration étant pressurisé par l'intermédiaire de l'orifice d'accès pneumatique ; et
un orifice de vidange (634) situé au niveau ou à proximité du fond de la chambre d'aspiration ;
la console chirurgicale possédant une source lumineuse linéaire pour projeter de la lumière dans la chambre d'aspiration et un système de réseau de capteurs linéaires (504) pour une détection en continu du niveau de fluide à l'intérieur de la chambre d'aspiration de la cassette, et comprenant un dispositif de commande (508) couplé avec le système de réseau de capteurs et le système d'aspiration destiné à gérer une pluralité de fonctions utilisant la chambre d'aspiration ;
la cassette étant adaptée pour être disposée à l'intérieur de la partie de réception de cassette de sorte que ladite surface de fluide dans la chambre d'aspiration (624) est mesurable par ledit système de réseau de capteurs (504) afin de fournir des informations sur la volumétrie et sur le flux d'aspiration à la console chirurgicale,
le dispositif de commande (508) étant adapté pour effectuer une opération de reflux et une opération d'amorçage d'une canalisation d'aspiration (602) reliant une pièce à main (576, 601) et l'orifice d'aspiration (632) de la cassette, l'orifice d'aspiration (632) et l'orifice de vidange (634) étant disposés dans la chambre d'aspiration de la cassette afin de permettre au fluide présent dans la chambre d'être complètement utilisé pour le reflux et l'amorçage, et la chambre d'aspiration étant configurée pour fonctionner comme un réservoir de fluide pour un reflux soutenu et un amorçage par propulsion sans perturber la détection de niveau en continu.

2. Console chirurgicale selon la revendication 1, comprenant en outre :
un système pneumatique (608) couplé de manière fluidique à l'orifice d'accès pneumatique (636) pour fournir une pression d'aspiration ou un vide (656) à la chambre d'aspiration.

3. Console chirurgicale selon la revendication 1, dans laquelle le dispositif de commande (508) peut être actionné pour gérer une vitesse d'écoulement du fluide d'aspiration sur la base de changements du niveau de fluide survenant au fil du temps dans la chambre d'aspiration.

4. Console chirurgicale selon l'une quelconque des revendications 1 à 3, comprenant en outre :
une pièce à main (576, 601) pour extraire le fluide d'aspiration depuis le site chirurgical pendant la procédure chirurgicale ; et
une conduite d'aspiration (602) reliant la pièce à main et l'orifice d'aspiration (632).

5. Cassette chirurgicale selon la revendication 4, dans laquelle le dispositif de commande (508) peut être actionné pour mener une opération de reflux soutenu, en forçant le liquide hors de la pièce à main (576, 601).

6. Console chirurgicale selon la revendication 4, dans laquelle le dispositif de commande (508) peut être actionné pour mener une opération d'amorçage pour la conduite d'aspiration (602).

7. Console chirurgicale selon la revendication 4, dans laquelle le dispositif de commande (508) peut être actionné pour mener une opération d'amorçage pour la conduite d'aspiration (602) en faisant en sorte que le système à vide fournisse de la pression à la chambre d'aspiration (604) et remplace l'air de la conduite d'aspiration par du liquide pressurisé en provenance de la chambre d'aspiration.

8. Console chirurgicale selon l'une quelconque des revendications 1 à 7, comprenant en outre :
une poche de vidange (626) destinée à contenir une partie du fluide d'aspiration ; et
un canal fluidique (630) connectant l'orifice de vidange et la poche de vidange.
